# EUROPÄISCHE PATENTSCHRIFT

(11) **EP 0 864 312 B1**
(45) Veröffentlichungstag und Bekanntmachung des Hinweises auf die Patenterteilung: **18.02.2004**
(21) Anmeldenummer: 98104271.6
(22) Anmeldetag: 10.03.1998
(51) Int. Cl.: A61K 6/093

(54) **Zwei-Komponenten-Kit und Stift auf Guttaperchabasis zur Verwendung mit dem Kit**
Two component kit and guttapercha post to be used in combination with the kit
Kit à deux composants et pivot en gutta-percha à utiliser avec ce kit

(30) Priorität: 10.03.1997 DE 19709531
(43) Veröffentlichungstag der Anmeldung: 16.09.1998
(73) Patentinhaber: Coltène/Whaledent GmbH + Co. KG, 89129 Langenau (DE)
(72) Erfinder: Engelbrecht, Jürgen, 22085 Hamburg (DE); Eberlein, Jürgen, 89122 Langenau (DE); Manschedel, Werner, 89122 Langenau (DE); Ziegler, Wolfram, 25335 Bokholt-Hanredder (DE)
(74) Vertreter: Forstmeyer, Dietmar, Dr. rer. nat., Dipl.-Chem.

(56) Entgegenhaltungen:
- DE-A- 3 743 983
- US-A- 3 082 526
- US-A- 4 449 938
- US-A- 5 165 893

## Beschreibung

Die Erfindung betrifft einen Kit mit zwei Komponenten, die nach Vermischen miteinander aushärten und als Wurzelfüllungsmaterial geeignet sind. Sie betrifft ferner einen Stift auf Guttaperchabasis als fakultative weitere Komponente für den Kit.

Aus EP-0 563 749 B1 ist bereits ein aus zwei Phasen bestehendes röntgenopakes Material zur Anfertigung von provisorischen Kronen und Brücken bekannt, wobei die Phasen
- aus einer Paste aus difunktionellen Acrylaten, Aktivatoren und röntgenopakem Füllstoff einerseits und
- aus nicht-polymerisierbarem (inertem) Weichmacher, Katalysator und Strukturbildner bestehen.

Der Einsatz dieses bekannten Materials als Wurzelfüllungsmaterial ist nicht vorgesehen.

DE 3 915 592 A1 beschreibt einen Dentalzement
aus einer Härtungsflüssigkeit, und zwar einem mit Carboxylgruppen modifizierten Silikonöl, und
einem Härtungsbeschleuniger, und zwar einem Metalloxid und/oder einem Metallhydroxid.

Dieser bekannte Dentalzement härtet also zu zementösen Massen aus, bei denen es sich um anorganische Salze des sauren Silikonöls handelt. Die Entfernung des ausgehärteten Dentalzements aus einem Wurzelkanal ist jedoch problematisch und kann zu einer unerwünschten Beschädigung des Wurzelkanals führen. In keinem Fall ist es möglich, den Dentalzement einstückig aus dem Kanal herauszuziehen.

Aus US 3 082 526 A ist es bekannt, eine Mischung zum Füllen von Wurzelkanälen einzusetzen, die
aus einem Diorganosiloxan mit endständigen Hydroxylgruppen der Formel HO(R₂SiO)ₙH und
einem Organotriacyloxisilan der Formel RSi(OAc)₃ besteht.

Die beiden Komponenten gehen unter Vernetzung eine Kondensationsreaktion ein, die jedoch infolge Abspaltung von HOAc zu einem unerwünschten Schrumpf führen kann.

Nach US 3 127 363 A kann
- ein Organosiloxan (A) der allgemeinen Formel XO-Si(R)₂-[O-SiR₂]ₙ-O-Si(R)₂-OX mit
- einem trifunktionellen Vernetzungsmittel (B) beispielsweise der Formel RSi(OH)₃ vernetzend kondensiert werden. Nach diesem Stand der Technik (Spalte 8 Zeile 43) kann die bekannte Zusammensetzung zum Verschließen von Randspalten verwendet werden, die ein Wurzelkanal und ein in den Kanal eingesetzter Stift miteinander bilden. Da sich dieser Stand der Technik einer Kondensationsreaktion bedient, kann das bereits angesprochene unerwünschte Schrumpfen nicht ausgeschlossen werden.

Dokument DE 37 43 983 betrifft ein Röntgenkontrast-Abdruckmaterial zur Kontrolle des Wurzelkanals, wobei als Silikonabdruckmassen Hydroxydimethylpolysiloxane mit Hydroxidgruppen an beiden Enden und Ortho- oder Polyethylsilikate mit einer Ethoxygruppe als Vernetzer oder Vinylpolymethylsiloxane mit einer Vinylendgruppe, Hydrogenpolymethylsiloxan mit aktivem Wasserstoff an beiden Enden und Additionspolymerisationskatalysatoren, wie Katalysatoren auf Platinbasis, eingesetzt werden. Derartige Zusammensetzungen reagieren jedoch entweder unter Abspaltung von Kondensationsprodukten, was zu den vorstehend beschriebenen Nachteilen führt, oder sie führen aufgrund des monofunktionellen Vinylpolymethylsiloxans mit einer Vinylendgruppe nur zu dimeren Verbindungen, die nicht vernetzt sind und leicht abgebaut werden können; für ein dauerhaftes Verbleiben im Zahn sind die Produkte nicht stabil genug. Insbesondere verbleibt in der Füllung ein Restmonomerengehalt, der aus der Füllung durch Gewebeflüssigkeit etc. ausgetragen werden kann. Dadurch kommt es zu Läsionen in der Wurzelfüllung und damit zu einer Bakterienbesiedlung, die zu einer Entzündung führen kann. Außerdem kann es zu einer lang anhaltenden Freisetzung von physiologisch unerwünschten Monomeren kommen.

Aufgabe der Erfindung ist es nun, ein Wurzelfüllungsmaterial vorzusehen, das bequem in einen freigelegten Wurzelkanal einzubringen ist, diesen gut abschließt, auch Randspalten gut abschließt, die vom Wurzelkanal und einem oder mehreren eingeführten Stiften gebildet werden, sowie Spalten, welche zwischen den Stiften gebildet werden, und das möglichst einstückig ohne Beschädigung des Wurzelkanals entfernt werden kann.

Es ist eine weitere Aufgabe der Erfindung, ein Wurzelfüllungsmaterial bereitzustellen, das für einen langen Zeitraum, zum Beispiel 10 bis 15 Jahre, im Zahn verbleiben kann, ohne daß Löcher auftreten oder physiologisch bedenkliche Stoffe freigesetzt werden.

Gemäß einer Ausführungsform der Erfindung wird dazu ein Kit vorgesehen, der zwei Komponenten umfaßt, die nach Vermischen miteinander aushärten und als Wurzelfüllungsmaterial geeignet sind, wobei
- eine der beiden Komponenten (Komponente I) aus einem oder mehreren Silikonölen besteht oder diese umfaßt, die mindestens zwei SiH-Gruppen aufweisen,
- die andere der beiden Komponenten (Komponente II) aus einem oder mehreren Silikonölen besteht oder diese umfaßt, die mindestens zwei Vinylgruppen aufweisen, und
- eine der beiden Komponenten zusätzlich einen Katalysator für eine Additionsreaktion oder eine Additionsvernetzung der beiden Komponenten I und II enthält.

Es hat sich gezeigt, daß sich mit dem erfindungsgemäßen Kit Wurzelkanalfüllungen vorsehen lassen, die leicht applizierbar sind, eine exzellente Randspaltdichtigkeit sowie Dichtigkeit zwischen den gegebenenfalls verwendeten Stiften bieten und sich bei Bedarf leicht als Ganzes entfernen lassen. Insbesondere können die erfindungsgemäßen Komponenten dreidimensional vernetzt werden, wobei keine Monomeren zurückbleiben. Derartige Materialien sind langzeit-stabil, d.h. sie können problemlos 10 oder sogar 15 Jahre im Wurzelkanal verbleiben, ohne daß es zu Läsionen in der Füllung oder einer Freisetzung von Monomeren etc. käme. Insbesondere sind die erfindungsgemäßen Füllmaterialien formstabil.

Zur Herstellung der Materialien werden erfindungsgemäß vernetzende Mischungen aus Vinylsilikonen und Hydrogensilikonen in dünnfließendem Zustand eingesetzt; zur Viskosität der Komponenten des erfindungsgemäßen Kits und ihrer Mischungen kann sich der Fachmann z.B. an die EP-0 563 749 B1 halten. Beispiele für selbstmischende Anmischsysteme sind ferner in EP-0 232 733 A1 und EP-0 261 466 A1 beschrieben; diese Anmischsysteme sind für additionsvernetzende Systeme (im Unterschied zu kondensationsvernetzenden Systemen) gut geeignet. Mit einem feinen Zusatzaufsatz auf das Ende des statischen Mischers kann das Einbringen des fertiggemischten Systems wesentlich erleichtert werden.

Der erfindungsgemäße Kit ist in einer bevorzugten Ausführungsform dadurch gekennzeichnet, daß das mindestens eine Silikonöl mit Vinylgruppen mindestens drei Vinylgruppen umfaßt und/oder daß das mindestens eine Silikonöl mit SiH-Gruppen mindestens drei SiH-Gruppen umfaßt. Durch geeignete Auswahl der jeweiligen Anzahl an funktionellen Gruppen können die Eigenschaften des Materials, wie Vernetzung, Härte, Elastizität etc. beliebig eingestellt werden.

Erfindungsgemäß kann das Silikonöl mit Vinylgruppen auch kleinere Mengen an monofunktionellen Silikonölen, insbesondere Vinylpolymethyldisiloxan, enthalten. Der Gehalt an Silikonölen mit nur einer Vinylgruppe sollte jedoch, bezogen auf die Gesamtmenge an Silikonölen mit Vinylgruppen, 5 Gew.-% nicht überschreiten. Vorzugsweise beträgt die Menge nicht mehr als 2 Gew.-%, am stärksten bevorzugt enthält die Komponente überhaupt kein Silikonöl mit nur einer Vinylgruppe.

Als Silikonöle mit mindestens zwei Vinylgruppen werden zum Beispiel Divinylpolymethyldisiloxan und/oder Trivinylpolymethylsiloxane eingesetzt.

Erfindungsgemäß kann das Silikonöl mit SiH-Gruppen auch kleinere Mengen an monofunktionellen Silikonölen enthalten. Der Gehalt an Silikonölen mit nur einer SiH-Gruppe sollte jedoch, bezogen auf die Gesamtmenge an Silikonölen mit SiH-Gruppen, 5 Gew.-% nicht überschreiten. Vorzugsweise beträgt die Menge nicht mehr als 2 Gew.-%, am stärksten bevorzugt enthält die Komponente überhaupt kein Silikonöl mit nur einer SiH-Gruppe.

Bei dem Silikonöl mit mindestens zwei SiH-Gruppen kann es sich erfindungsgemäß z.B. um Hydrogenpolymethyldisiloxan handeln.

Als Katalysator kann man eine platinhaltige Verbindung verwenden, beispielsweise Hexachloroplatinsäure.

Komponente I und II des erfindungsgemäßen Kits können übliche Hilfsmittel umfassen, insbesondere
- inerte Füllstoffe, insbesondere inerte anorganische Füllstoffe, wie Quarz oder Aluminiumoxid,
- mindestens ein Röntgenkontrastmittel, ausgewählt aus der Gruppe der Zink-, Ytterbium-, Yttrium-, Gadolinium-, Zirkonium-, Strontium-, Wolfram-, Tantal-, Niob-, Barium-, Wismut-, Molybdän- und Lanthanpulver, pulvrigen Legierungen davon, Oxiden, Fluoriden, Sulfaten, Carbonaten, Wolframaten und Carbiden davon,
- ionenspendende Füllstoffe,
- thixotropierende Füllstoffe, vorzugsweise Kieselsäure,
- plastifizierende Zusätze, vorzugsweise Silikonöl und/oder Paraffinöl,
   und/oder
- desinfizierende und/oder devitalisierende Zusätze.

Die erfindungsgemäß einsetzbaren Röntgenkontrastmittel können eine Korngröße von 0,1 bis 50 Mikrometer, bevorzugt von 0,3 bis 40 Mikrometer, stärker bevorzugt von 0,5 bis 30 Mikrometer, noch stärker bevorzugt von 0,8 bis 20 Mikrometer, und am stärksten bevorzugt von 1 bis 10 Mikrometer aufweisen. Wird das Korn zu groß, so wird das Material zu grobkörnig und kann gegebenenfalls nicht mehr ohne weiteres in den Wurzelkanal eingebracht werden. Wird das Korn zu fein, so wird seine Oberfläche zu groß, was ebenfalls zu Problemen führen kann.

Bei den ionenspendenden Füllstoffen kann es sich erfindungsgemäß um calcium-, zink-, carbonat-, fluorid- und/oder phosphathaltige Zusätze handeln. Beispiele für calciumhaltige Zuschlagstoffe sind Calciumcarbonat, Hydroxylapatit, Tricalciumphosphat und Tetracalciumphosphat. Als zinkhaltiger Zusatz kann Zinkoxid zum Einsatz kommen. Auch Gläser können als ionenspendende Füllstoffe verwendet werden.

Für die thixotropierenden Füllstoffe kann auf amorphe Siliciumdioxidmodifikationen verwiesen werden, beispielsweise auf pyrogene und gefällte Kieselsäure sowie Kieselgur. Besonders geeignet sind agglomerierte pyrogene Kieselsäuren oder gesintertes Kieselgel, wie sie in EP-0 040 232 und EP-0 113 926 beschrieben sind. Diese Füllstoffe neigen wenig zum Nachdicken und haben die Eigenschaft, daß ihr thixotropes Verhalten gleichmäßig ist und kaum davon abhängig ist, wie rasch die Verarbeitung erfolgt.

Erfindungsgemäß ist es zweckmäßig, die beiden Komponenten I und II des Kits in einem Verhältnis von 10:90 bis zu 90:10, vorzugsweise in einem Verhältnis von 40:60 bis zu 60:40, und am stärksten bevorzugt zu etwa gleichen Teilen auf Volumenbasis vorzusehen. Ein derartiges Verhältnis ist besonders dann vorteilhaft, wenn man mit selbstmischenden Anmischsystemen gemäß EP-0 232 733 oder EP-0 261 466 arbeitet. Die Komponenten des erfindungsgemässen Kits können dazu in getrennten Kartuschen vorgesehen sein.

Gemäß einer weiteren Ausführungsform der Erfindung werden ein Stift oder mehrere Stifte auf Isoprenbasis als fakultative weitere Komponente des erfindungsgemäßen Kits vorgesehen, insbesondere auf Basis von Trans-Poly-Isopren, Guttapercha oder Balata, wobei der Stift mit einem erfindungsgemäßen Silikonöl imprägniert sein kann, das SiH-Gruppen aufweist.

Für einen derartigen Stift kann man von einer Primerlösung ausgehen, die das Hydrogensilikon enthält, das mit ungesättigten Gruppen des Stifts chemisch reagiert und eine feste Verbindung eingeht. Als Silikonöl mit SiH-Gruppen kann man wiederum Hydrogenpolymethyldisiloxan verwenden.

Wenn man einen erfindungsgemäß imprägnierten Stift oder mehrere derartige Stifte in einen freigelegten Wurzelkanal einführt und den Randspalt mit Hilfe eines erfindungsgemäßen Kits verschließt, verbindet sich der Stift mit dem ausgehärteten Wurzelfüllungsmaterial derart, daß sich der Stift später leicht als Ganzes zusammen mit dem Kit entfernen läßt.

Natürlich ist es auch möglich, erfindungsgemäß einen Stift vorzusehen, der mit einem erfindungsgemäßen Silikonöl mit SiH-Gruppen im Gemisch mit mindestens einem erfindungsgemäßen Silikonöl mit Vinylgruppen imprägniert ist. Bei diesem mindestens einem Silikonöl mit Vinylgruppen kann es sich wiederum maximal um geringe Mengen an monofunktionellem Silikonöl, wie Vinylpolymethyldisiloxan, in Kombination mit einer Hauptmenge an einem polyfunktionellen Silikonöl handeln, insbesondere um ein bifunktionelles Silikonöl wie Divinylpolymethyldisiloxan, oder ein polyfunktionelles Silikonöl.

Nachstehend wird die Erfindung anhand von Beispielen näher beschrieben.

### Beispiel 1: Herstellung des Wurzelfüllungsmaterials auf Basis additionsvernetzender Silikone.

Man stellt eine Mischung A aus folgenden Bestandteilen her:

| | |
|---|---|
| Trivinylpolymethyldisiloxan | 66 Teile |
| Vinylpolymethyldisiloxan | 2 Teile |
| Hydrogenpolymethyldisiloxan mit 2 SiH-Gruppen | 15 Teile |
| Paraffinöl | 12 Teile |
| Pyrogene Kieselsäure | 5 Teile |

Nach inniger Vermischung wird evakuiert. Außerdem wird eine Mischung B mit folgenden Bestandteilen hergestellt:

| | |
|---|---|
| Trivinylpolymethyldisiloxan | 68 Teile |
| Silikonöl | 27 Teile |
| Hexachloroplatinsäure (Katalysator) | 0,001 Teil |
| Pyrogene Kieselsäure | 5 Teile |

Nach inniger Vermischung wird ebenfalls evakuiert.

Mischung A und Mischung B werden zu gleichen Teilen in eine Doppelkartusche mit statischem Mischer gegeben und direkt in einen vorbereiteten Wurzelkanal appliziert oder man taucht eine Guttaperchaspitze in diese Mischung und bringt diese so in den Wurzelkanal ein. Innerhalb von 10 min ist die Gesamtmischung ausgehärtet. Die erfindungsgemäße Silikonfüllung dichtet randspaltenfrei ab.

### Beispiel 2: Herstellung und Anwendung eines Primers für Guttaperchastifte

Man stellt eine Lösung aus den folgenden Bestandteilen her:

| | |
|---|---|
| Hydrogenpolymethyldisiloxan (Primer) | |
| mit 2 SiH-Gruppen | 10 Teile |
| Toluol | 90 Teile |

Die Lösung wird auf einen Guttaperchastift aufgetragen und getrocknet. Nach dem Einbringen eines Gemischs der Mischungen A und B gemäß Beispiel 1 in einen vorbereiteten Wurzelkanal wird der Guttaperchastift in den Wurzelkanal eingebracht. Nach dem Aushärten der erfindungsgemäßen Wurzelkanalfüllmasse ist der Guttaperchastift mit der Masse voll verbunden. Der Guttaperchastift kann samt Wurzelfüllungsmaterial leicht aus dem Wurzelkanal herausgezogen werden.

## Patentansprüche

1. Kit umfassend zwei Komponenten, die nach Vermischen miteinander aushärten und als Wurzelfüllungsmaterial geeignet sind, wobei
- eine der beiden Komponenten (Komponente I) aus einem oder mehreren Silikonölen besteht oder diese umfaßt, die mindestens zwei SiH-Gruppen aufweisen,
- die andere der beiden Komponenten (Komponente II) aus einem oder mehreren Silikonölen besteht oder diese umfaßt, die mindestens zwei Vinylgruppen aufweisen, und
- eine der beiden Komponenten zusätzlich einen Katalysator für eine Additionsreaktion oder Additionsvernetzung der beiden Komponenten I und II enthält.

2. Kit nach Anspruch 1, **dadurch gekennzeichnet, daß** das mindestens eine Silikonöl mit Vinylgruppen mindestens drei Vinylgruppen umfaßt.

3. Kit nach Anspruch 1 oder 2, **dadurch gekennzeichnet, daß** das mindestens eine Silikonöl mit SiH-Gruppen mindestens drei SiH-Gruppen umfaßt.

4. Kit nach einem der vorhergehenden Ansprüche, **gekennzeichnet durch** ein Gemisch aus bis zu 5 Gew.-% monofunktionellen und mindestens 95 Gew.-% polyfunktionellen Silikonölen mit Vinylgruppen.

5. Kit nach einem der vorhergehenden Ansprüche, **gekennzeichnet durch** ein Gemisch aus bis zu 5 Gew.-% monofunktionellen und mindestens 95 Gew.-% polyfunktionellen Silikonölen mit Si-H-Gruppen.

6. Kit nach einem der vorhergehenden Ansprüche, **gekennzeichnet durch** Hydrogenpolymethyldisiloxan als Silikonöl mit SiH-Gruppen.

7. Kit nach einem der vorhergehenden Ansprüche, **gekennzeichnet durch** einen platinhaltigen Katalysator, insbesondere Hexachloroplatinsäure.

8. Kit nach einem der vorhergehenden Ansprüche, **gekennzeichnet durch** übliche Hilfsmittel, insbesondere
- inerte Füllstoffe, insbesondere inerte anorganische Füllstoffe, vorzugsweise Quarz oder Aluminiumoxid,
- mindestens ein Röntgenkontrastmittel, ausgewählt aus der Gruppe der Zink-, Ytterbium-, Yttrium-, Gadolinium-, Zirkonium-, Strontium-, Wolfram-, Tantal-, Niob-, Barium-, Wismut-, Molybdän- und Lanthanpulver, pulvrigen Legierungen davon, Oxiden, Fluoriden, Sulfaten, Carbonaten, Wolframaten und Carbiden davon,
- Glasionomer-Pulver,
- plastifizierende Zusätze, vorzugsweise Silikonöl und/oder Paraffinöl,
- thixotropierende Füllstoffe, vorzugsweise Kieselsäure,
- ionenspendende Füllstoffe und/oder
- desinfizierende und/oder devitalisierende Zusätze.

9. Kit nach Anspruch 8, **gekennzeichnet durch** calcium-, zink-, carbonat-, fluorid- und/oder phosphathaltige Zusätze als anorganische Träger.

10. Kit nach einem der vorhergehenden Ansprüche, **dadurch gekennzeichnet, daß** die Komponenten I und II des Kits zu etwa gleichen Teilen (Volumenbasis) vorgesehen sind.

11. Kit nach einem der vorhergehenden Ansprüche, **dadurch gekennzeichnet, daß** die Komponenten des Kits in getrennten Kartuschen vorgesehen sind.

12. Kit nach Anspruch 11, **dadurch gekennzeichnet, daß** die Kartuschen in ein statisches Mischapplikationsgerät einsetzbar sind.

13. Kit gemäß einem der vorhergehenden Anspüche enthaltend als weitere Komponente einen Stift auf Isoprenbasis, **dadurch gekennzeichnet, daß** der Stift mit mindestens einem Silikonöl nach einem der vorstehenden Anspruche imprägniert ist, das SiH-Gruppen aufweist.

14. Kit nach Anspruch 13, **dadurch gekennzeichnet, daß** der Stift mit mindestens einem Silikonöl, das SiH-Gruppen aufweist, im Gemisch mit mindestens einem Silikonöl nach einem der vorstehenden Ansprüche imprägniert ist, das Vinylgruppen aufweist.

## Claims

1. Kit including two components which harden upon mixing with one another and are suited as root-filling material, wherein
- one of the two components (Component I) consists of or comprises one or more silicone oils having at least two SiH groups,
- the other of the two components (Component II) consists of or comprises one or more silicone oils having at least two vinyl groups, and
- one of the two components additionally contains a catalyst for an addition reaction or addition cross-linking of the two components I and II.

2. Kit according to Claim 1, **characterised in that** the at least one silicone oil with vinyl groups includes at least three vinyl groups.

3. Kit according to Claim 1 or 2, **characterised in that** the at least one silicone oil with SiH groups includes at least three SiH groups.

4. Kit according to any one of the preceding claims,
**characterised by** a mixture of up to 5 wt.-% monofunctional and at least 95 wt.-% poly-functional silicone oils with vinyl groups.

5. Kit according to any one of the preceding claims,
**characterised by** a mixture of up to 5 wt.-% monofunctional and at least 95 wt.-% polyfunctional silicone oils with SiH groups.

6. Kit according to any one of the preceding claims,
**characterised by** hydrogen polymethyldisiloxane as silicone oil with SiH groups.

7. Kit according to any one of the preceding claims,
**characterised by** a platinum-containing catalyst, in particular hexachloroplatinic acid.

8. Kit according to any one of the preceding claims,
**characterised by** usual additives, in particular,
- inert fillers, in particular inert inorganic fillers, preferably quartz or aluminium oxide,
- at least one X-ray contrast agent, selected from the group of zinc, ytterbium, yttrium, gadolinium, zirconium, strontium, wolfram, tantalum, niobium, barium, bismuth, molybdenum and lanthanum powder, powdered alloys thereof, oxides, fluorides, sulfates, carbonates, wolframates, and carbides thereof,
- glasionomer powder,
- plastifying agents, particularly silicone oil and/or paraffin oil,
- thixotropic fillers, preferably silicic acid
- ion-donor fillers, and/or
- disinfecting and/or devitalizing agents.

9. Kit according to Claim 8, **characterised by** calcium, zinc, carbonate, fluoride and/or phosphate-containing agents as inorganic carrier.

10. Kit according to anyone of the preceding claims,
**characterised in that** the components I and II of the kit are provided in substantially equal amounts (volume basis).

11. Kit according to any one of the preceding claims,
**characterised in that** the components of the kit are provided in separate cartridges.

12. Kit according to Claim 11, **characterised in that** the cartridges are insertable in a static mixing application device.

13. Kit according to any of the preceding claims containing as further component a pin on the basis of isoprene,
**characterised in that** the pin is impregnated with at least one silicone oil according to any one of the preceding claims having SiH groups.

14. Kit according to Claim 13, **characterised in that** the pin is impregnated with at least one silicone oil having SiH groups mixed with at least one silicone oil according to any one of the preceding claims having vinyl groups.

## Revendications

1. Equipement englobant deux constituants qui durcissent après mélange et qui conviennent comme matériau de remplissage de racine, l'un des deux constituants (constituant I) étant constitué d'une ou plusieurs huiles de silicone qui présentent au moins deux groupes SiH, ou bien comprenant de telles huiles, l'autre des deux constituants (constituant II) étant constitué d'une ou plusieurs huiles de silicone qui présentent au moins deux groupes vinyle, ou bien comprenant de telles huiles, et l'un des deux constituants contenant en plus un catalyseur pour une réaction d'addition ou une réticulation par addition des deux constituants I et II.

2. Equipement selon la revendication 1, **caractérisé en ce que** la au moins une huile de silicone avec des groupes vinyle comprend au moins trois groupes vinyle.

3. Equipement selon la revendication 1 ou 2, **caractérisé en ce que** la au moins une huile de silicone avec des groupes SiH comprend au moins trois groupes SiH.

4. Equipement selon l'une quelconque des revendications précédentes, **caractérisé par** un mélange de jusqu'à 5% en poids d'huiles de silicone monofonctionnelles et d'au moins 95% en poids d'huiles de silicone polyfonctionnelles avec des groupes vinyle.

5. Equipement selon l'une quelconque des revendications précédentes, **caractérisé par** un mélange de jusqu'à 5% en poids d'huiles de silicone monofonctionnelles et d'au moins 95% en poids d'huiles de silicone polyfonctionnelles avec des groupes SiH.

6. Equipement selon l'une quelconque des revendications précédentes, **caractérisé par** de l'hydrogénopolyméthyldisiloxane en tant qu' huile de silicone ayant des groupes SiH.

7. Equipement selon l'une quelconque des revendications précédentes, **caractérisé par** un catalyseur contenant du platine, en particulier de l'acide hexachloroplatinique.

8. Equipement selon l'une quelconque des revendications précédentes, **caractérisé par** des adjuvants habituels, en particulier :
- des charges inertes, en particulier des charges minérales inertes, de préférence du quartz ou de l'oxyde d'aluminium,
- au moins un agent de contraste pour la radiographie, choisi parmi les poudres de zinc, d'ytterbium, d'yttrium, de gadolinium, de zirconium, de strontium, de tungstène, de tantale, de niobium, de baryum, de bismuth, de molybdène et de lanthane, les alliages pulvérulents des éléments précédents, et les oxydes, fluorures, sulfates, carbonates, tungstates et carbures des éléments précédents,
- de la poudre de verre ionomère,
- des additifs plastifiants, de préférence de l'huile de silicone et/ou de l'huile de paraffine,
- des charges thixotropes, de préférence de l'acide silicique,
- des charges donnant des ions, et/ou
- des additifs désinfectants et/ou dévitalisants.

9. Equipement selon la revendication 8, **caractérisé par** des additifs contenant du calcium, du zinc, du carbonate, du fluorure et/ou du phosphate en tant que support minéral.

10. Equipement selon l'une quelconque des revendications précédentes, **caractérisé en ce que** les constituants I et II de l'équipement sont prévus en des proportions en volume à peu près identiques.

11. Equipement selon l'une quelconque des revendications précédentes, **caractérisé en ce que** les constituants de l'équipement sont prévus dans des cartouches séparées.

12. Equipement selon la revendication 11, **caractérisé en ce que** les cartouches sont utilisables dans un appareil d'application de mélange statique.

13. Equipement selon l'une quelconque des revendications précédentes, comprenant comme autre constituant un pivot à base d'isoprène, **caractérisé en ce que** le pivot est imprégné d'au moins une huile de silicone selon l'une quelconque des revendications précédentes, qui présente des groupes SiH.

14. Equipement selon la revendication 13, **caractérisé en ce que** le pivot est imprégné d'au moins une huile de silicone présentant des groupes SiH, en mélange avec au moins une huile de silicone présentant des groupes vinyle selon l'une quelconque des revendications précédentes.
